# EUROPEAN PATENT APPLICATION

(11) **EP 4 793 350 A1**
(43) Date of publication of application: **19.08.2026**
(21) Application number: 25382122.7
(22) Date of filing: 12.02.2025
(51) Int. Cl.: C12N 9/48

(54) **FUSION PROTEINS AND THE USE THEREOF FOR THE TREATMENT OF CORONAVIRUS INFECTIONS**

(71) Applicant: Fundació Privada Institut de Recerca Sobre Immunopatologies-Caixa Irsicaixa, 08916 Badalona, Barcelona (ES); Barcelona Supercomputing Center-Centro Nacional de Supercomputación, 08034 Barcelona (ES); Universidad Autònoma de Barcelona, 08913 Cerdanyola del Valles, Barcelona (ES); Institut de Recerca i Tecnologia Agroalimentaries (IRTA), 08140 Caldes de Montbui (Barcelona) (ES); Fundació Institut d'Investigació en Ciències de la Salut Germans Trias i Pujol (IGTP), 08916 Badalona (ES)
(72) Inventor: BLANCO ARBUÉS, Julián M., E-08916 Badalona, Barcelona (ES); SERGE TRINITÉ, Benjamin, E-08916 Badalona, Barcelona (ES); ABANCO ESPUGA, Ferrán, E-08916 Badalona, Barcelona (ES); TARRÉS FREIXAS, Ferrán, E-08916 Badalona, Barcelona (ES); IZQUIERDO USEROS, Nuria, E-08916 Badalona, Barcelona (ES); CARRILLO MOLINA, Jorge, E-08916 Badalona, Barcelona (ES); LEPORE, Rosalba, E-08034 Barcelona (ES); SEGALÉS COMA, Joaquim, E-08193 Bellaterra, Barcelona (ES); VERGARA ALERT, Júlia, E-08193 Bellaterra, Barcelona (ES); MODREGO GUILLÉN, Andrea, E-28049 Madrid (ES)
(74) Representative: ABG Intellectual Property Law, S.L.

(57) **Abstract**

The present invention relates to fusion proteins comprising a mutated peptidase domain of angiotensin converting enzyme 2 and a constant domain of an immunoglobulin, as well as nucleic acids encoding the fusion proteins, which are useful in the treatment of coronavirus infection.

## Description

### BACKGROUND

The COVID-19 pandemic has fueled more than 770 million cases and killed more than 6.9 million people worldwide. The rapid development and distribution of vaccines was key to containing the pandemic and limiting casualties; however, not everybody can take full advantage of these vaccines. This is especially the case for immunocompromised individuals and part of the elderly population who are at higher risk of developing severe COVID-19. Immunocompromised are a varied group, which represents about 2-3% of the overall population and include people with human immunodeficiency virus (HIV) infection, primary immunodeficiencies, cancers, transplants, and people treated with immunosuppressive biologics and medications. Therefore, there is a need for alternative prophylactic and therapeutic options. Neutralizing monoclonal antibodies provide such an option. Indeed SARS-CoV-2 specific neutralizing monoclonals have been temporarily approved as prophylactic and therapeutic treatments for individuals with an elevated risk of developing severe disease. They helped decrease hospital stay, disease severity and death in the most fragile infected individuals. However, since the beginning of the pandemic and due to a massive transmission rate, SARS-CoV-2 has been rapidly evolving, modulating fitness, pathogenicity, and its capacity to escape both innate and adaptive immunity. In particular, the omicron family of variants, which display a highly mutated spike, were associated with a sharp increase in infection cases and notably breakthrough infections. Unsurprisingly, omicron variants were also associated with the failure of most therapeutic monoclonal antibodies available. While new monoclonal antibodies continue to be isolated and validated which can neutralize Omicron subvariants, this appears to be a never-ending and costly race, and an important lag remains between new variants' occurrence and the availability of effective antibodies.

The main receptor of SARS-CoV-2 is the angiotensin converting enzyme 2 (ACE2) protein recognized by the viral Spike. Interestingly, soluble forms of ACE2 can act as a decoy receptor and neutralize SARS-CoV-2. In addition, many coronaviruses circulating in animal reservoirs and with zoonotic potential, are likely to target human ACE2 as a viral receptor (Wang Q et al. Communications Biology, volume 6, 1051 (2023)). Therefore, there is a need for new ACE2 based biologics that serve as first line emergency treatment for future pandemics.

### SUMMARY OF THE INVENTION

A first aspect of the present invention is a fusion protein comprising the peptidase domain of angiotensin converting enzyme 2 (ACE2) and a constant domain of an immunoglobulin, wherein ACE2 comprises mutations T27F, K31W, S43M and Q81S, and at least one additional mutation that inactivates ACE2 catalytic activity. These proteins are herein referred to as the proteins of the invention and these mutations are referred herein as the mutations of the invention or M1 or mutations of the invention.

A second aspect of the present invention is a nucleic acid encoding the fusion proteins of the first aspect.

A third aspect of the present invention is the fusion proteins of the first aspect or the nucleic acids of the second aspect, for use in the treatment of coronavirus infection.

### DETAILED DESCRIPTION OF THE INVENTION

The inventors have found new ACE2-Fc molecules with a particular set of mutations that have improved coronavirus neutralization potency and are effective against all the variants tested, even the newest SARS-CoV-2 variants.

In a preferred embodiment of the first aspect of the present invention, the fusion protein comprises additional mutations are H345A and H505A. These mutations prevent the catalytic activity of the ACE2 peptidase domain.

In a preferred embodiment of the first aspect of the present invention, both the ACE2 peptidase domain and the constant domain of an immunoglobulin are of humans. In a preferred embodiment of the first aspect of the present invention, the constant domain of an immunoglobulin is SEQ ID NO: 13. Association of the ACE2 peptidase domain with an Fc region allows to create dimeric proteins, it increases the plasma half-life, and it confers Fc-receptor-dependent immune functionalities which are required for therapeutic activity against infection, such as coronavirus, e.g. SARS-CoV-2 infection. In an embodiment, the fusion protein comprises the peptidase domain of human ACE2 or of any homolog or ortholog thereof, or a fragment thereof that retains the ability of binding to the coronavirus. The peptidase domain of ACE2 or truncated form thereof can effectively neutralize a virus that uses the ACE2 as a host-binding receptor. The virus may comprise SARS-CoV, HCoV-NL63 or SARS-CoV2. The constant domain of an immunoglobulin can be derived from IgG1, IgG2, IgG3 or IgG4. Preferably, the constant domain of an immunoglobulin is that of IgG1.

In a preferred embodiment of the first aspect of the present invention, the peptidase domain of ACE2 is preceded by a signal peptide. Preferably, the signal peptide is the natural signal peptide of human ACE2. More preferably, this peptide is SEQ ID NO: 14.

In a preferred embodiment of the first aspect of the present invention, the fusion protein comprises the extracellular domain of ACE2. The extracellular domain of ACE2 includes the natural signal peptide (aa 1-17) and the peptidase domain of ACE 2 (aa 18-615).

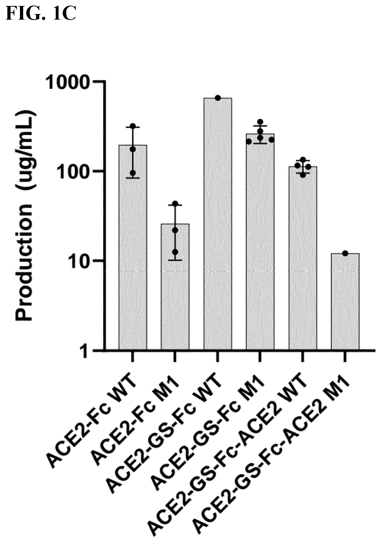

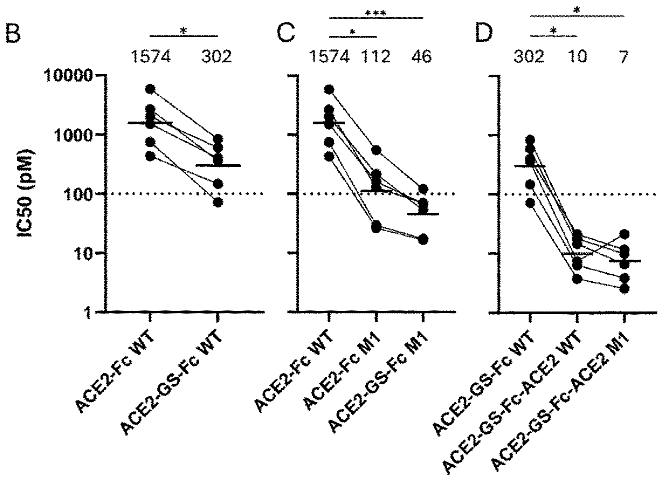

In a preferred embodiment of the first aspect of the present invention, the fusion protein comprises a linker between the peptidase domain of ACE2 and the constant domain of an immunoglobulin. In a preferred embodiment, the linker is flexible. Preferably, it has SEQ ID NO: 15. The presence of the linker has a double advantage: it highly improves the protein expression efficiency, as can be seen in Fig. 1 C and it also highly improves the neutralizing activity, as can be seen in Figs. 2 B, C. This is especially relevant for the proteins of the invention, since their expression is affected by the mutations of the invention in comparison with the unmutated, wild type (WT) ACE2 sequence. In terms of neutralizing activity, the inventors have surprisingly found a synergic effect of the mutations of the invention and the flexible linker, reaching up to 36-fold improvement in neutralizing activity.

In a preferred embodiment of the first aspect of the present invention, the fusion protein comprises more than one peptidase domain of ACE2. Preferably, the fusion protein comprises two peptidase domains of ACE2. In a preferred embodiment, the fusion protein has one ACE2 peptidase domain located amino terminally of the constant domain of an immunoglobulin and the second ACE2 peptidase domain located carboxy terminally of the constant domain of an immunoglobulin. Preferred embodiments of the first aspect of the present invention are those having SEQ ID NO: 3, SEQ ID NO: 7 or SEQ ID NO: 11. The tetrameric structure of the protein of the invention provides an improved neutralizing activity, as can be seen in Fig. 2D.

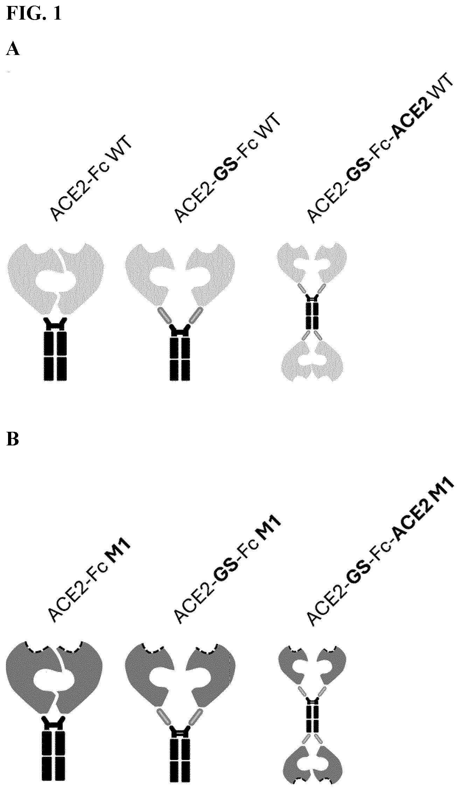

The fusion protein of SEQ ID NO: 3 comprises the structure ACE2-Fc M1 shown in Fig. 1B. The fusion protein of SEQ ID NO: 7 comprises the structure ACE2-GS-Fc M1 shown in Fig. 1B. The fusion protein of SEQ ID NO: 11 comprises the structure ACE2-GS-Fc-ACE2 M1 shown in Fig. 1B.

The ACE2-Fc proteins of the present invention form dimers. When the fusion protein comprises two ACE2 peptidase domains, tetrameric structures are formed (Fig. 1B).

In a preferred embodiment of the first aspect, the fusion proteins are isolated.

In a preferred embodiment of the second aspect of the present invention, the nucleic acid has SEQ ID NO: 4, SEQ ID NO: 8 or SEQ ID NO: 12. In another preferred embodiment of the second aspect, the nucleic acid is an expression vector. The nucleic acids of SEQ ID NO: 4, SEQ ID NO: 8 or SEQ ID NO: 12 codify for fusion proteins with SEQ ID NO: 3, SEQ ID NO: 7 or SEQ ID NO: 11, respectively. In a preferred embodiment of the second aspect, the nucleic acids are isolated.

In a preferred embodiment of the third aspect of the present invention, the fusion protein of the invention is used in the treatment of any coronavirus infection. In a preferred embodiment it is used in the treatment of SARS-CoV-2.

Another aspect of the present invention refers to a cell that comprises the nucleic acid of the second aspect of the invention. In a preferred embodiment, the cell line is eukaryotic. The cell line may include, but is not limited to, CHO, 293 and a Vero and a cell line derived therefrom, e.g., Vero E6 cells or HEK293T cells.

Another aspect of the present invention refers to a method for preparing the fusion proteins of the first aspect of the invention, comprising the following steps:
(a) transfecting a cell line with the expression vector of the second aspect to obtain a cell line expressing the fusion protein of the first aspect;
(b) culturing the cell line obtained in step (a) under a culture condition to produce the fusion protein of the first aspect; and
(c) purifying the fusion protein produced in step (b).

Another aspect of the present invention refers to a pharmaceutical composition comprising the fusion proteins of the first aspect and a pharmaceutically acceptable carrier.

Another aspect of the present invention refers to the use of the fusion proteins of the first aspect in the preparation of a medicament for treating or preventing an ACE2-related disease. The disease may be a disease selected from any disease caused by an infection of a virus employing ACE2 as a receptor. The virus may comprise a coronavirus, e.g., SARS-CoV, HCoV-NL63, or SARS-CoV2. The disease may be selected from pneumonia, severe acute respiratory infection, renal failure, heart failure, adult respiratory distress syndrome (ARDS), liver injury, intestinal disease, or severe acute respiratory syndrome. The fusion proteins of the first aspect can be used for administration in emergency situations, thereby avoiding high morbidity and lethality caused by the infection of viruses employing ACE2 as a receptor, especially coronavirus infections. The fusion proteins of the first aspect can also be used for passive immunization of a person at risk of exposure to a virus, especially a coronavirus, employing ACE2 as a receptor.

In any of the aspects of the invention, the present invention refers also to the embodiments where the expression "comprises" or "comprising" means "consists of" or "consisting of". The present invention includes any combination of the different embodiments and preferred embodiments disclosed herein for any of the aspects of the invention.

### DESCRIPTION OF THE FIGURES

**Figure 1****: ACE2-Fc design and production.** A. Schematic representation of the general structural modifications of the ACE2-Fc WT molecule. B. Schematic representation of the different structures of the ACE2-Fc molecules. GS: flexible linker. WT: Wild type. M1: mutated according to the invention. C: Expression efficiency of the different molecules: 4 days after transient transfection in Expi293F cells, the supernatants were harvested and concentration of the ACE2-Fc molecules was measured by ELISA against human IgG Fc.

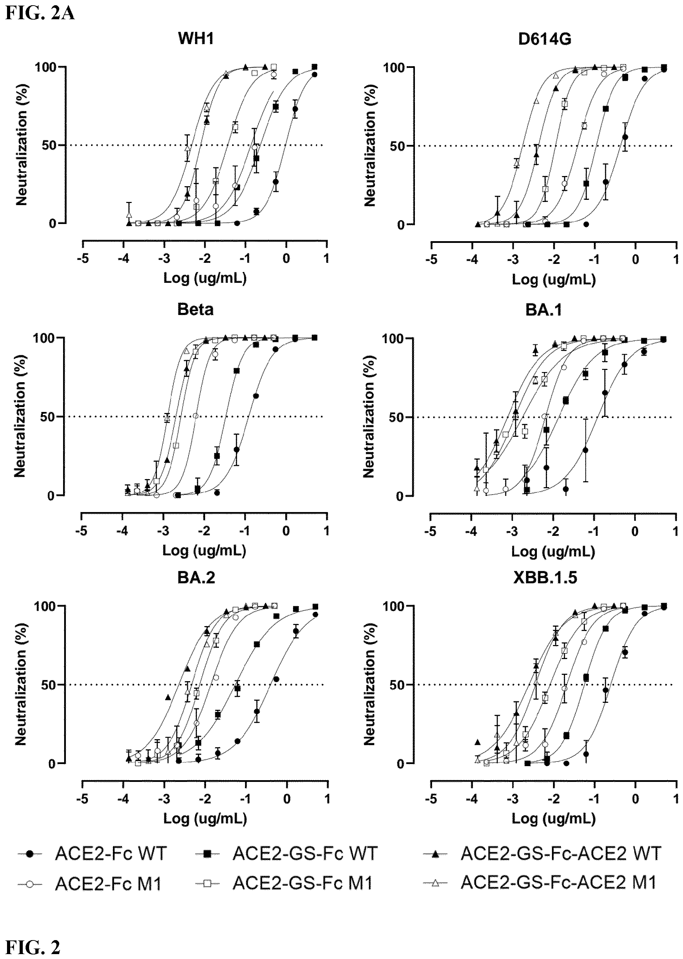

**Figure 2****: Neutralizing activity of modified ACE2-Fc molecules.** The new ACE2-Fc molecules neutralizing activity was tested against a panel of 6 Spike variants (WH1, D614G, BETA, BA.1, BA.2, XBB.1.5). A. Neutralization profiles. B. Effect of the flexible linker on the neutralizing activity. C. Effect of the mutations and flexible linker on the neutralizing activity. D. Effect of the tetrameric structure and mutations on the neutralizing activity. Horizontal bars and top numbers indicate IC50 geomeans across the variant tested.

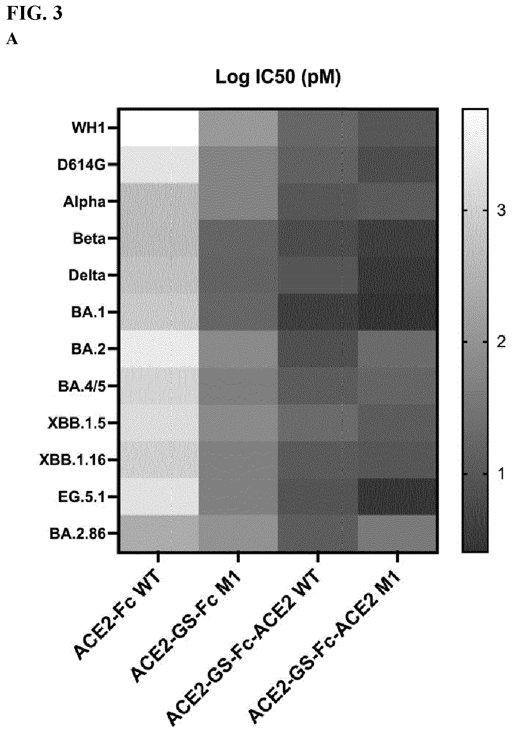

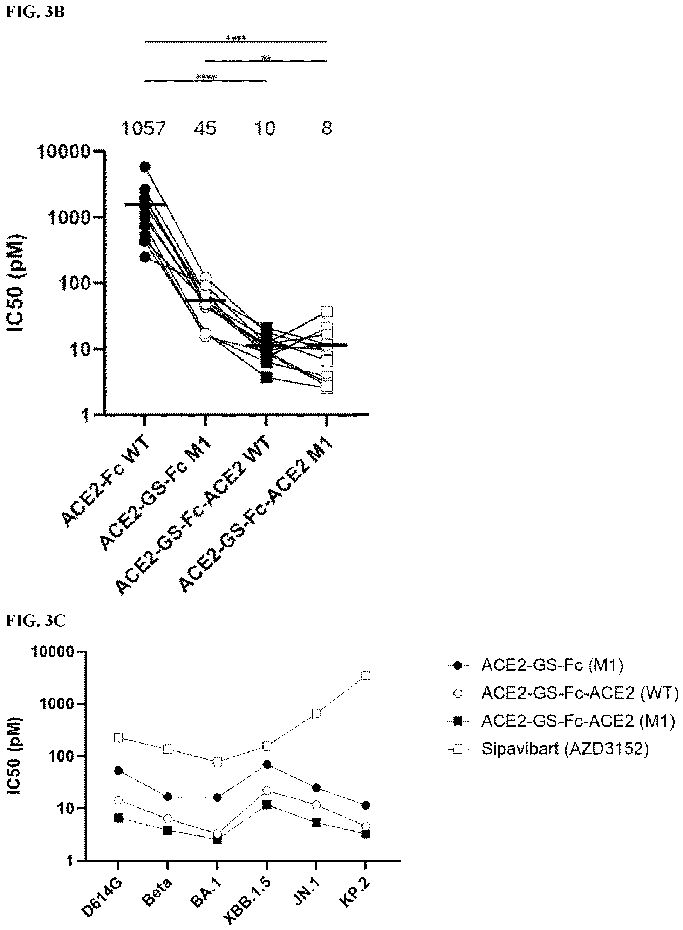

**Figure 3****: Neutralizing activity of the new ACE2-Fc molecules.** A/B. Neutralizing activity of ACE2-Fc molecules of the invention against a large panel of Spike variants (indicated) represented as a heatmap (A) and dot chart (B). Horizontal bars and top numbers indicate IC50 geomeans across the variant tested. C. Comparison of the neutralizing activity of the new ACE2-Fc molecules of the invention and Sepavibart against the indicated variants.

### EXAMPLES

### 1. New fusion protein generation

We first generated an ACE2-Fc fusion protein composed of the ACE2 peptidase domain, including the natural signal peptide (a.a. 1 to 615) directly fused to the constant region of a human IgG1 Fc (ACE2-Fc WT; SEQ ID NO: 1 and SEQ ID NO: 2; Fig. 1A.). The ACE2 domain included 2-point mutations, H345A and H505A, for catalytic activity inactivation. From this initial template, we performed several modifications to improve the production and activity of the protein. To verify that Fc bound ACE2 elements had optimal access to the Spike Receptor Binding Domain (RBD), we generated alternative molecules that included a (G₃S)₁₀ flexible linker (G₃S)₁₀ between the N-terminal ACE2 and Fc domains (ACE2-GS-Fc WT; SEQ ID NO: 5, SEQ ID NO: 6, Fig. 1A). To increase the global avidity of the molecule, we designed a tetrameric fusion protein. To this end, a second ACE2 peptidase domain was fused at the C-terminal end of the Fc region behind a (G₃S)10 flexible linker (ACE2-GS-Fc-ACE2 WT; SEQ ID NO: 9, SEQ ID NO: 10; Figure 1A). Finally, to enhance stability and binding affinity to the RBD, in silico site-saturation mutagenesis was performed on the ACE2-RBD 3D complex. 3D models of the SARS-CoV-2 RBD were generated for the primary circulating viral variants. These variants included key mutations: i) N501Y (observed in the UK variant, B.1.1.248 (Brazil/Japan), and B.1.351 (South Africa)), ii) K417N (B.1.351), K417T (B.1.1.248), and iii) E484K (B.1.351 and B.1.1.248). The analysis identified four positions including residues at the ACE2-RBD interface (T27, K31) and additional sites (S43, Q81). The T27F mutation was predicted to introduce new intermolecular interactions with aromatic residues Y489 and F456 on the RBD, common to all variants. The K31W mutation was predicted to form new stacking interactions with the RBD residue Y489 and intramolecular interactions within the ACE2 domain. Additionally, mutation of Q81 to S was predicted to enhance the stability of the ACE2 by forming a new hydrogen bond within the alpha-helix, particularly with main-chain atoms of residue S77. In contrast, the S43M mutation was expected to contribute mainly through van der Waals interactions with neighboring residues ACE2 F40 and S44. We combined all 4 mutations to create the new ACE2-Fc of the invention. The structural modifications, including the addition of a flexible linker and the design of the tetrameric molecule were also applied to the new ACE2-Fc mutant (SEQ ID NO: 3, SEQ ID NO: 4, SEQ ID NO: 7, SEQ ID NO: 8, SEQ ID NO: 11, SEQ ID NO: 12, Fig. 1B).

### 2. Production of the fusion proteins

All protein candidates were expressed in the Expi293F cells system. The protein concentration in the supernatants was quantified by an anti-human Fc ELISA (Fig. 1C). The WT ACE2-Fc molecule showed a mean concentration of 196.8 µg/mL. M1 mutations reduced the overall production efficiency (26 µg/mL; 7.6-fold reduction) whereas the addition of the flexible linker was highly beneficial and substantially increased the production of both WT (657 µg/mL; 3.3-fold increase) and M1 (261.9 µg/mL; 10-fold increase) fusion proteins. The tetrameric proteins were less efficiently expressed but the yield was still above 10 microg/mL. All the proteins were purified by protein A and size-exclusion and quantified by optical density before functional validation. The protein purification quality of each molecule was confirmed by SDS-page Coomassie stain in reduced conditions showing a main band at the expected molecular weight for each molecule.

The neutralizing activity of all the fusion proteins was first measured against a panel of 6 pseudoviruses expressing relevant pre-Omicron (WH1, D614G and Beta) and Omicron derived (BA.1, BA.2 and XBB.1.5) Spike variants (Fig. 2). The neutralizing activity of the WT ACE2-Fc molecule ranged from 5,852 pM (WH1) to 431 pM (BETA). In comparison ACE2-GS-Fc consistently showed increased activity ranging from 835 pM (WH1) to 72 pM (BA.1) (5.8-fold average improvement) showing that the presence of the flexible linker improved the access of the ACE2 domains to Spikes. The addition of the four point mutations of the invention in the binding interface also greatly improved the activity of the molecule, ranging from 551 pM (WH1) to 26 pM (Beta) (15-fold average improvement) and synergized with the flexible linker to raise the neutralizing activity further, ranging from 122 pM (WH1) to 16.7 pM (BETA) (36-fold average improvement). On the other side, the tetrameric proteins showed the greatest activity with a neutralizing activity ranging from 21.1 pM (XBB.1.5) to 3.7 pM (BA.1) for the WT ACE2 (33-fold improvement compared to ACE2-GS Fc WT; Fig. 2) and ranging from 21.3 pM (BA.2) to 2.6 pM (BA.1) for the M1 ACE2 (6.8-folds improvement compared to ACE2-GS-Fc M1).

The control wild-type ACE2-Fc and the structures that demonstrated IC50 below 100 pM, (ACE2-GS-Fc M1 and both wild-type and M1 ACE2-GS-Fc-ACE2) were further tested against a larger panel of SARS CoV-2 pseudoviruses (WH1, D614G, Alpha, Beta, Delta, BA.1, BA.2, BA.4/5, XBB.1.5, XBB.1.16, EG.5.1 and BA.2.86). The dimeric mutant lowered the geomean activity to 45.09 pM, 23-fold more potent than the basic wild-type ACE2-Fc. Geomean IC50s were further lowered to 10.12 pM and 8.20 pM when neutralizing with WT and M1 tetramers, respectively.

Finally, we compared the neutralizing activity of our molecules to Sipavibart (AZD3152), which is a broad neutralizing antibody against SARS-CoV-2 under phase III trial (Supernova) as a prophylactic strategy for immunocompromised population. The neutralizing activity of Sipavibart was tested and compared to ACE2-GS-Fc M1, ACE2-GS-Fc-ACE2 WT and ACE2-GS-Fc-ACE2 M1 against D614G, Beta, BA.1, XBB.1.5, JN.1 and KP.2 variants. The fusion proteins neutralized all the variants with an IC50 below 100 pM, including the more recent JN.1 (25.0, 11.6, and 5.3 pM, respectively) and KP.2 (11.3, 4.6, and 3.28 pM, respectively). On the other hand, Sipavibart only had a high neutralization activity against BA.1 (77.1 pM) and (as reported elsewhere) was not able to neutralize the KP.2 variant (>3455.7 pM) (Figure 3C).

### 3. Methods

### Molecular design

All Ace2-Fc constructs were built based on the natural ACE2 signal peptide (aa: 1-17) followed by the ACE2 peptidase domain (aa:18-615; Uniprot.org entry number Q9BYF1) including the point mutations H345A and H505A for catalytical inactivation, and the constant region (CH2-CH3) of the human IgG1 heavy chain (Fc; aa: 218-449; Uniprot.org entry number P0DOX5). Optionally, a flexible linker (G₃S)₁₀ was introduced between the ACE2 peptidase domain and the Fc region. For the generation of a tetrameric protein, a second ACE2 peptidase domain was fused in the C-terminal of the Fc, separated by a (G₃S)₁₀ flexible linker. All the elements were synthetized (Geneart) and final constructions were generated by subcloning. Notably, the flexible linker was added to the N-terminal end of the Fc by subcloning via an Alel site located in the ACE2 sequence and BamHI site located in the Fc region. The final constructs were all inserted into pcDNA3.4 via Hindlll and Xhol sites.

### In Silico Mutagenesis

3D models of the ACE2-RBD complexes were constructed using the SWISS-MODEL homology modeling server, using the structure PDB ID: 6LZG as initial template PMID: 29788355. In silico site-saturation mutagenesis was performed with FoldX PMID: 15980494.

### Protein production

Recombinant fusion proteins were produced in Expi293F cells according to the protocol defined by the manufacturer (Expi293 Expression System, ThermoFisher Scientific). After 4 days, the supernatant was centrifuged for 10 minutes at 3000xg and filtered using a 0.22 um PES membrane (Stericup-GP Sterile Vacuum Filtration System, Millipore). The filtered supernatant was stored at -80°C.

### Affinity chromatography

Recombinant fusion proteins were purified through Protein A since they contain the human IgG1 Fc. The filtered supernatant obtained after transfection was thawed in a 37°C water bath and subsequently purified by affinity chromatography using protein A columns (HiTrap MabSelect PrismA, Cytiva) in an ÄKTA Go protein purification system (Cytiva). These assays were performed using PBS 1X as equilibration buffer, glycine 0.1 M (pH = 3.5) as elution buffer and Tris 1 M (pH = 8) as neutralizing buffer. The eluate was further concentrated through a two-step size exclusion via centrifugal filters (Amicon Ultra-15 PLTK membrane 30 kDa, Millipore) spined at 3000xg for 25 and 15 minutes. The concentrated product was filtered through a 0.22 um PVDF membrane (Millipore), quantified by optical density based on its extinction coefficient and then stored at -80°C.

### Human Fc ELISA

Non-purified transfection supernatant was first analyzed using a sandwich ELISA in 96- well plates. The assay was based on an anti-human F(ab')₂ fragment as a capture antibody (AffiniPure^{™} F(ab')₂ Fragment Goat Anti-Human IgG) and a HRP-conjugated anti-human F(ab')₂ fragment as a secondary antibody (Peroxidase AffiniPure^{™} F(ab')₂ Fragment Goat Anti-Human IgG, Jackson ImmunoResearch). Plates were coated overnight at 4°C and blocked in 1X PBS + 1% BSA + 0.05% Tween-20 for 2 hours at room temperature. Samples were added and incubated overnight at 4°C, followed by optical measurement at 492 and 620 nm wavelengths (Microplate Reader Ensight, Perkin) after the addition of the o-phenylenediamine dydrochloride (OPD) substrate solution.

### Protein SDS page and Coomassie stain

Purified and concentrated fusion proteins were evaluated in Western blot and Coomassie staining. Samples were heat-shocked (5 minutes at 95°C) under reducing conditions and run in polyacrylamide gel (NuPAGE Bis-Tris 4-12%, Invitrogen). Afterwards, proteins were transferred to a PVDF membrane (Trans-Blot Turbo Mini 0.2 µm PVDF Transfer Packs, BioRad), which was blocked and incubated with a HRP-conjugated anti human F(ab')₂ fragment (Peroxidase AffiniPure^{™} F(ab')₂ Fragment Goat Anti-Human IgG, Jackson ImmunoResearch). The reading was performed adding the chemiluminescent substrate (SuperSignal^{™} West Pico PLUS Chemiluminescent Substrate, Thermo Scientific) onto the membrane and viewed in an ChemiDoc Imaging System (BioRad). For Coomassie staining, the gel was directly washed twice with deionized water for 10 minutes before and after staining it with SimplyBlue SafeStain (Invitrogen) for 1 hour. Gel images were captured also by ChemiDoc Imaging System (BioRad).

### Pseudovirus generation

HIV reporter pseudo viruses expressing SARS-CoV-2 S protein and Luciferase were generated. pNL4-3.Luc.R-.E- was obtained from the National Institute of Health (NIH) acquired immune deficiency syndrome (AIDS) Reagent Program. SARS-CoV-2.SctΔ19 was generated (GeneArt) from the full protein sequence of the original WH1 SARS-CoV-2 Spike (Genbank: MN908947.3) with a deletion of the last 19 amino acids in C-terminal, human-codon optimized and inserted into pcDNA3.1(+). A similar procedure was followed to generate expression plasmids for the Delta, BA.1, BA.2 and BA.4/5 variants of SARS CoV-2 S protein according to consensus data (www.outbreak.info/). Expi293F cells were transfected with pNL4-3.Luc.R-.E- and the different SARS-CoV-2.SctΔ19 Spike plasmids using ExpiFectamine 293 Reagent (Thermo Fisher Scientific). Control pseudo viruses were obtained by replacing the S protein expression plasmid with a VSV-G protein (Vesicular Stomatitis Virus G protein) expression plasmid. Supernatants were harvested 48 hours after transfection, filtered at 0.45 µm, frozen, and titrated on HEK293T cells overexpressing WT human ACE-2 (Integral Molecular, USA). Serial 1/2 dilutions of the supernatant were prepared in quadruplicate in a 96-well plate using DMEM medium supplemented with 10% fetal bovine serum (D10). Then, 10,000 293T cells, constitutively expressing ACE2 on their surface, and dextran were added. The co-culture was incubated at 37°C for 2 days. After incubation, 100 uL of medium was removed, and 50 uL of Britelite Plus (Revvity), a luciferase substrate was added to quantify the activity of the luciferase from infected 293T cells which was expressed in relative light units (RLUs). Luminescence was measured with the Microplate Reader Ensight (Perkin).

### Pseudovirus based neutralization assay

100 uL of fusion proteins at known concentrations were added in duplicate wells, followed by serial 1/3 dilutions. Subsequently, 50 uL luciferase reporter SARS-CoV-2 pseudoviruses were added also in each well, targeting 20,000 RLU based on prior titration. The co-culture was incubated at 37°C for 45 minutes. Meanwhile, ACE2-expressing 293T cells were washed with 1X PBS and harvested using Versene solution (Gibco). A total of 10,000 293T cells were added to each well in DMEM medium supplemented with 10% fetal bovine serum (FBS) and 0.1% dextran. After three days of incubation at 37°C, 150 uL of medium were removed from each well, and 50 uL of Britelite Plus (Revvity) were added to measure luciferase expression. Luminescence was quantified by luminometry using a Microplate Reader Ensight (Perkin). The values were normalized, and the IC50 (concentration inhibiting 50% of the infection) was calculated by plotting and fitting all duplicate neutralization values and the log of the fusion protein concentration to a 4-parameters equation in Prism 9.0.2 (GraphPad Software, USA). This assay has been previously validated with a replicative viral inhibition assay (Tinité, B et al. Sci Rep. 2021 Jan 28;11(1):2608).

## Claims

1. A fusion protein comprising the peptidase domain of angiotensin converting enzyme 2 (ACE2) and a constant domain of an immunoglobulin, wherein ACE2 comprises mutations T27F, K31W, S43M and Q81S, and at least one additional mutation that inactivates ACE2 catalytic activity.

2. The fusion protein of claim 1, wherein the additional mutations are H345A and H505A.

3. The fusion protein of any one of claims 1 or 2, wherein the peptidase domain of ACE2 is preceded by a signal peptide.

4. The fusion protein of claim 3, wherein the signal peptide is SEQ ID NO: 14.

5. The fusion protein of any one of claims 1 to 4, comprising the extracellular domain of ACE2.

6. The fusion protein of any one of claims 1 to 5, comprising a linker between the peptidase domain of ACE2 and the constant domain of an immunoglobulin.

7. The fusion protein of claim 6, wherein the linker has SEQ ID NO: 15.

8. The fusion protein of any one of claims 1 to 7, wherein the constant domain of an immunoglobulin is SEQ ID NO: 13.

9. The fusion protein of any one of claims 1 to 8, comprising more than one peptidase domain of ACE2.

10. The fusion protein of any one of claims 1 to 9, comprising two peptidase domains of ACE2.

11. The fusion protein of claim 10, wherein one ACE2 peptidase domain is located amino terminally of the constant domain of an immunoglobulin and the second ACE2 peptidase domain is located carboxy terminally of the constant domain of an immunoglobulin.

12. The fusion protein of any one of claims 1 to 11, having SEQ ID NO: 3, SEQ ID NO: 7 or SEQ ID NO: 11.

13. A nucleic acid encoding the fusion proteins of any one of claims 1 to 12.

14. The nucleic acid of claim 13, having SEQ ID NO: 4, SEQ ID NO: 8 or SEQ ID NO: 12.

15. The fusion proteins of any one of claims 1 to 12 or the nucleic acids of any one of claims 13 or 14, for use in the treatment of coronavirus infection.
